(19) Europäisches Patentamt
European Patent Office
Office européen des brevets

(11) Veröffentlichungsnummer: **0 056 461**
**B1**

(12) # EUROPÄISCHE PATENTSCHRIFT

(45) Veröffentlichungstag der Patentschrift: **07.03.84**

(51) Int. Cl.³: **C 07 D 211/22**, A 01 N 43/40

(21) Anmeldenummer: **81110348.0**

(22) Anmeldetag: **11.12.81**

(54) **N-3'-(p-tertiär-Butylphenyl)-2'-methyl-propyl-1'-piperidin-derivate, diese enthaltende Fungizide und Verfahren zur Bekämpfung von Pilzen mit diesen Verbindungen.**

(30) Priorität: **16.01.81 DE 3101233**

(43) Veröffentlichungstag der Anmeldung: **28.07.82 Patentblatt 82/30**

(45) Bekanntmachung des Hinweises auf die Patenterteilung: **07.03.84 Patentblatt 84/10**

(84) Benannte Vertragsstaaten: **AT BE CH DE FR GB IT LI LU NL SE**

(56) Entgegenhaltungen:
**EP - A - 0 000 333**
**EP - A - 0 006 992**
**DE - A - 2 752 096**
**DE - A - 2 752 135**
**GB - A - 888 657**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Himmele, Walter, Dr., Eichenweg 14, D-6909 Walldorf (DE)**
Erfinder: **Pommer, Ernst-Heinrich, Dr., Berliner Platz 7, D-6703 Limburgerhof (DE)**

**EP 0 056 461 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-3′-(p-tertiär-Butylphenyl)-2′-methyl-propyl-1′-piperidinderivate, diese enthaltende Fungizide und Verfahren zur Bekämpfung von Pilzen mit diesen Verbindungen

Die vorliegende Erfindung betrifft wertvolle N-3′-(p-tertiär-Butylphenyl)-2′-methyl-propyl-1′-piperidinderivate und ihre Salze mit guter fungizider Wirkung, Fungizide, die diese Verbindungen enthalten, und Verfahren zur Bekämpfung von Pilzen mit diesen Verbindungen.

Es ist bekannt, N-3′-(p-tertiär-Butylphenyl)-2′-methyl-propyl-1′-3-hydroxymethylpiperidin als Fungizid zu verwenden (DE-OS 27 27 482). Es ist ferner bekannt, das N-3′-(p-tertiär-Butylphenyl)-2′-methylpropyl-1′-piperidin (DE-OS 27 52 135) und das entsprechende -4-hydroxypiperidin als Fungizide zu verwenden (DE-OS 27 27 482).

Es wurde gefunden, dass N-3′-(p-tertiär-Butylphenyl)-2′-methyl-propyl-1′-piperidinderivate der Formel

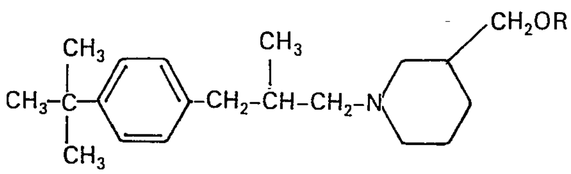

in der R einen Acetyl- oder Propionylrest bedeutet, und ihre Salze eine gute fungizide Wirkung haben, die der Wirkung der bekannten Verbindung überlegen ist.

Salze sind beispielsweise die Salze mit anorganischen oder organischen Säuren, z.B. Chloride, Fluoride, Bromide, Jodide, Sulfate, Nitrate, Phosphate, Acetate, Propionate oder mit Säuren von Tensiden, z.B. Dodecylbenzolsulfonsäure.

Die Herstellung erfolgt beispielsweise durch Umsetzung von 3-Hydroxymethyl-piperidin mit 3-p-tertiär-Butyl-phenyl-2-methyl-propanal in Gegenwart eines Reduktionsmittels, z.B. Ameisensäure bei Temperaturen von 50 bis 110°C und Veresterung der Hydroxyverbindung mit Acetanhydrid oder Propionsäureanhydrid in üblicher Weise.

Die folgenden Beispiele erläutern die Herstellung der neuen Verbindungen.

Die Ausgangsverbindung kann wie folgt hergestellt werden.

Vorschrift 1

Synthese von N-3′-(p-tert. Butylphenyl)-2′-methyl-propyl-1′-3-hydroxymethyl-piperidin

In einem 1-l-Kolben werden 204 g 3-(4′-tert.Butyl-phenyl-1′)-2-methylpropanal vorgelegt und unter Rühren 115 g 3-Hydroxymethylpiperidin zugegeben. Unter Kühlung werden anschliessend 50 g 98%ige Ameisensäure zugefügt. Der Ansatz wird anschliessend zum Sieden erhitzt, dabei tritt eine starke $CO_2$-Bildung auf. Nach 12stündigem Erhitzen auf 120 bis 140°C ist die Synthese beendet.

Zur Reinigung des Produktes wird bei einem Druck von 3 mbar das Produkt destilliert. Nach einem Vorlauf von 44 g, der bis zu einer Temperatur von 169°/3 mbar übergeht und nach der gaschromatographischen (GC) Analyse 27% des Endproduktes enthält, gehen 260 g Endprodukt zwischen 169 bis 174°C über. Nach der GC-Analyse liegt ein circa 95%iges Produkt vor. Die Ausbeute bezogen auf die beiden Einsatzprodukte beträgt 84%. Die NMR-Analyse zeigt die zu erwartenden Signale.

Beispiel 1

Synthese von N-3′-(p-tert.Butylphenyl)-2′-methyl-propyl-1′-3-acetoxymethyl-piperidin (I)

In einem 250-ml-Rundkolben werden 30 g N-3′-(p-tert.Butyl-phenyl)-2′-methyl-propyl-1′-3-hydroxymethylpiperidin vorgelegt und 50 g Acetanhydrid zugegeben. Als Katalysator für die Veresterung werden 1 ml Pyridin zugetropft. Zur vollständigen Umsetzung wird das Reaktionsgemisch 20 Minuten am Rückfluss erhitzt. Zur Reinigung des Produkts I wird bei 3 mbar das Reaktionsgemisch destilliert. Bis zu einer Temperatur von 153°C gehen 10 g (56%ig laut GC (Gaschromatographie)-Analyse). Die Hauptmenge des Produktes I geht zwischen 153 bis 155°C über. Nach der GC-Analyse liegt ein 99%iges Produkt vor. Die NMR-Analyse zeigt die zu erwartenden Signale.

| | | | | |
|---|---|---|---|---|
| ber: | C 76,16% | H 7,99% | O 8,45% | N 7,40% |
| gef: | C 76,0 % | H 7,9 % | O 8,9% | N 7,3 % |

Beispiel 2

Synthese von N-3′-(p-tert.Butylphenyl)-2′-methyl-propyl-1′-3-propionoxymethyl-piperidin (II)

Durch Umsetzung von 30 g N-3′-(p-tertiär-Butylphenyl)-2′-methyl-propyl-1′-3-hydroxymethylpiperidin mit 60 g Propionsäureanhydrid in Gegenwart von katalytischen Mengen von Pyridin (1 ml) wird das Produkt II hergestellt. Die Reinigung erfolgt gleichfalls durch Destillation bei einem Druck von 3 mbar. Es gehen im Temperaturbereich zwischen 169 bis 172°C bei 3 mbar 24 g Produkt II über. Nach der GC-Analyse liegt ein über 98%iges Produkt vor. Die NMR-Analyse liefert die zu erwartenden Signale.

| | | | | |
|---|---|---|---|---|
| ber: | 76,03% | H 10,37% | O 8,90% | N 3,90% |
| gef: | 76,5 % | H 10,2 % | O 9,20% | N 3,9 % |

Die erfindungsgemässen Wirkstoffe und die entsprechenden Fungizide sind insbesondere geeignet zur Bekämpfung von Pflanzenkrankheiten, z.B. Erysiphe graminis (echter Mehltau) an Getreide, Erysiphe cichoracearum (echter Mehltau) an Kürbisgewächsen, Podosphaera leucotricha an Äpfeln, Uncinula necator an Reben, Erysiphe polygoni an Bohnen, Sphaerotheca pannosa an Rosen, Microsphaera querci an Eichen, Botrytis cinerea an Erdbeeren, Reben, Mycosphaerella muscola an Bananen, Puccinia-Arten (Rostpilze) an Getreide, Uromyces appendiculatus und u.phaseoli an Bohnen, Hemileia vastatrix an Kaffee und Rhizoctonia solani. Sie sind systemisch

wirksam; sie werden sowohl über die Wurzel als auch über die Blätter aufgenommen und im Pflanzengewebe transportiert.

Bei der Anwendung des neuen Wirkstoffes zur Behandlung von Pflanzen gegen Pilzinfektionen liegen die Aufwandmengen zwischen 0,025 und 5 kg Wirkstoff/ha Fläche. Zum Oberflächenschutz von Bäumen oder Früchten kann der Wirkstoff auch in Verbindung mit Kunststoffdispersionen 0,25%ig bis 5%ig, bezogen auf das Gewicht der Dispersionen, verwendet werden. Die Fungizide enthalten im allgemeinen 0,1 bis 95 Gewichtsprozent Wirkstoff, vorzugsweise zwischen 0,5 und 90%. Der Wirkstoff kann mit anderen bekannten Fungiziden gemischt werden. In vielen Fällen erhält man dabei eine Vergrösserung des fungiziden Wirkungsspektrums; bei einer Anzahl von Fugizidmischungen in den Gewichtsverhältnissen 1:10 bis 10:1 treten auch synergistische Effekte auf, d.h. die fungizide Wirksamkeit des Kombinationsproduktes ist grösser als die der addierten Wirksamkeit der Einzelkomponenten. Wirkstoffe für solche Mischungen sind beispielsweise:

Dithiocarbamate und deren Derivate, wie Zinkdimethyldithiocarbamat, Manganäthylenbisdithiocarbamat, Mangan-Zink-äthylendiamin-bisdithiocarbamat, Zinkäthylenbisdithiocarbamat, Tetramethylthiuramdisulfid, Ammoniak-Komplex von Zink-(N,N-äthylen-bis-dithiocarbamat) und N,N'-Polyäthylen-bis-(thiocarbamoyl)-disulfid, Zink-(N,N'-propylen-bis-dithiocarbamat), Ammoniak-Komplex von Zink-(N,N'-propylen-bis dithiocarbamat) und N,N'-Polypropylen-bis-(thiocarbamoyl)-disulfid; heterocyclische Verbindungen, wie N-Trichlormethylthio-tetrahydrophthalimid, N-Trichlormethylthio-phthalimid, N-(1,1,2,2-Tetrachloräthylthio)-tetrahydrophthalimid, 1-(Butylcarbamoyl) -2-benzimidazol-carbaminsäuremethylester, 2-Methyloxycarbonylamino-benzimidazol, 2,3-Dihydro-5-carboxanilido-6-methyl-1,4-oxathiin-4,4-dioxid, 2,3-Dihydro-5-carboxanilido-6-methyl-1,4-oxathiin, 5-Butyl-2-dimethylamino-4-hydroxy-6-methyl-pyrimidin, 1,2-Bis-(3-äthoxycarbonyl-2-thioureido)-benzol, 1,2-Bis-(3-methoxycarbonyl-2-thioureido)-benzol und verschiedene andere Fungizide, wie Dodecylguanidinacetat, N-Dichlorfluormethylthio-N',N'-dimethyl-N-phenyl-schwefelsäurediamid, 2,5-Dimethyl-furan-3-carbonsäureanilid, 2,5-Dimethyl-furan-3-carbonsäure-cyclohexylamid, 2-Jodbenzoesäureanilid, 2-Brombenzoesäureanilid, 3-Nitro-isophtalsäure-diisopropylester, 1-(1,2,4-Triazolyl-1-1')-1-(4'-chlorphenoxy)-3,3-dimethyl-butan-2-on, 1-(1-Imidazolyl)-2-allyloxy-2-(2,4-dichlorphenyl)-äthan, Piperazin-1,4-diyl-bis- 1-(2,2,2-trichloräthyl- 1)-formamid, 2,4,5,6-Tetrachlor-isophthalonitril, 1,2-Dimethyl-3,5-diphenyl-pyrazoliniummethylsulfat.

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen, Beizen oder Giessen. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon, Wasser usw. in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl der Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind. An oberflächenaktiven Stoffen sind zu nennen:

Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexandecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octyl-phenoläther, äthoxyliertes Isooctylphenol-, Octylphenol-, Nonylphenol, Alkylphenolpolyglykoläther. Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs-und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kalk, Kreide, Talkum, Bolus, Löss, Ton, Dolomit, Diatemeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nusschalenmehl, Cellulosepulver und andere feste Trägerstoffe.

Zu den Mischungen oder Einzelwirkstoffen können Öle verschiedenen Typs, Herbizide, Fungizide, Nematozide, Insektizide, Bakterizide, Spurenelemente, Düngemittel, Antischaummittel (z.b. Silikone), Wachstumsregulatoren, Antidotmittel oder andere wirksame Verbindungen zugesetzt werden.

Für die folgenden Versuche wurden folgende bekannte Vergleichssubstanzen verwendet.

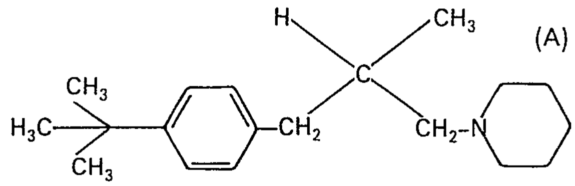

(A)

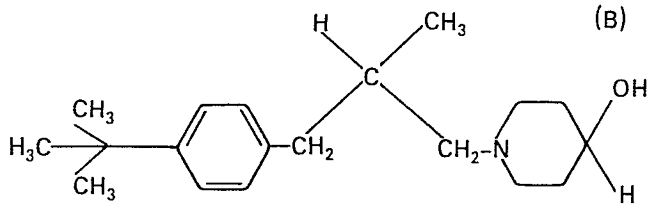

(B)

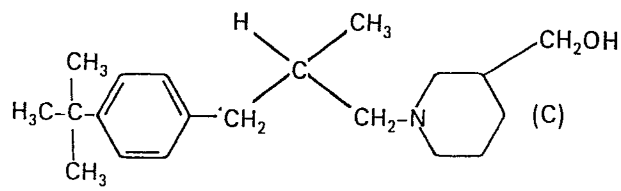

(C)

Wirksamkeit gegen Weizenmehltau

Blätter von in Töpfen gewachsenen Weizenkeimlingen der Sorte «Jubilar» werden mit wässrigen Emulsionen aus 80% (Gew.-%) Wirkstoff und 20% Emulgiermittel (bezogen auf die Trokkensubstanz) besprüht und nach dem Antrocknen des Spritzbelages mit Oidien (Sporen) des Weizenmehltaus (Erysiphe graminis var.tritici) bestäubt. Die Versuchspflanzen werden anschliessend im Gewächshaus bei Temperaturen zwischen 20 und 22°C und 75 bis 80% relativer Luftfeuchtigkeit aufgestellt. Nach 10 Tagen wird das Ausmass der Mehltauentwicklung ermittelt.

In diesem Versuch zeigten die neuen Wirkstoffe I und II bei Anwendung von 0,012; 0,006; 0,003 und 0,0015%igen Wirkstoffbrühen eine bessere fungizide Wirkung als der bekannte Wirkstoff A.

Blätter von in Töpfen gewachsenen Weizenpflanzen der Sorte «Jubilar» werden mit Sporen des Weizenbraunrostes (Puccinis recondita) künstlich infiziert und 48 Stunden lang bei 20 bis 25°C in einer wasserdampfgesättigten Kammer aufgestellt. Danach werden die Pflanzen mit wässrigen Spritzbrühen, die in dem Wasser gelöst oder emulgiert eine Mischung aus 80% des zu prüfenden Wirkstoffes und 20% Natriumligninsulfonat (bezogen auf die Trockensubstanz) enthalten, besprüht und im Gewächshaus bei Temperaturen zwischen 20 und 22°C und bei 75 bis 80% relativer Luftfeuchtigkeit aufgestellt. Nach 10 Tagen wird das Ausmass der Rostpilzentwicklung beurteilt.

In diesem Versuch zeigten die neuen Wirkstoffe I und II bei Anwendung von 0,025; 0,012 und 0,006%igen Wirkstoffbrühen eine bessere fungizide Wirkung als die bekannten Wirkstoffe A, B und C.

Beispiel a

Man vermischt 90 Gewichtsteile der Verbindung I mit 10 Gewichtsteilen N-Methyl-alphapyrrolidon und erhält eine Lösung, die zur Anwendung in Form kleinster Tropfen geeignet ist.

Beispiel b

10 Gewichtsteile der Verbindung II werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-mono-äthanolamid, 2 Gewichtsteilen Calciumsalz der Dodecylbenzolsulfonsäure und 2 Gewichtsteilen des Anlagerungsproduktes von 40 Mol Äthylenoxid an 1 Mol Ricinusöl besteht.

Durch Ausgiessen und feines Verteilen der Lösung erhält man eine wässrige Dispersion.

Beispiel c

20 Gewichtsteile der Verbindung I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingiessen und feines Verteilen der Lösung in Wasser erhält man eine wässrige Dispersion.

Beispiel d

20 Gewichtsteile der Verbindung II werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingiessen und feines Verteilen der Lösung in Wasser erhält man eine wässrige Dispersion.

Beispiel e

80 Gewichtsteile des Wirkstoffs I werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

Beispiel f

3 Gewichtsteile der Verbindung II werden mit 97 Gewichtsteilen feinteiligem Kaolin innig vermischt. Man erhält auf diese Weise ein Stäubemittel, das 3 Gewichtsprozent des Wirkstoffs enthält.

Beispiel g

30 Gewichtsteile der Verbindung I werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel h

40 Gewichtsteile des Wirkstoffs II werden mit 10 Teilen Natriumsalz eines Phenolsulfonsäureharnstoff-formaldehyd-kondensats, 2 Teilen Kieselgel und 48 Teilen Wasser innig vermischt. Man erhält eine stabile wässrige Dispersion. Durch Verdünnen mit Wasser erhält man eine wässrige Dispersion.

**Patentansprüche für die Vertragsstaaten: BE, CH, DE, FR, GB, IT, LI, LU, NL, SE**

1. N-3′-(p-tertiär-Butylphenyl)-2′-methyl-propyl-1′-piperidinderivat der allgemeinen Formel

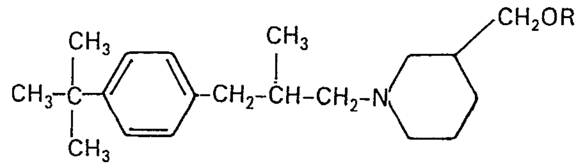

in der R einen Acetyl- oder Propionylrest bedeutet, und ihre Salze.

2. Fungizid enthaltend ein N-3′-(p-tertiär-Butylphenyl)-2′-methyl-propyl-1′-piperidinderivat der allgemeinen Formel

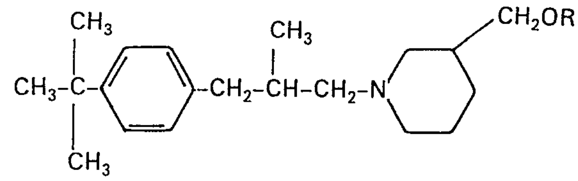

in der R einen Acetyl- oder Propionylrest bedeutet, und ihre Salze.

3. Fungizid enthaltend einen festen oder flüssigen Trägerstoff und ein N-3′-(p-tertiär-Butylphenyl)-2′-methyl-propyl-1′-piperidinderivat der allgemeinen Formel

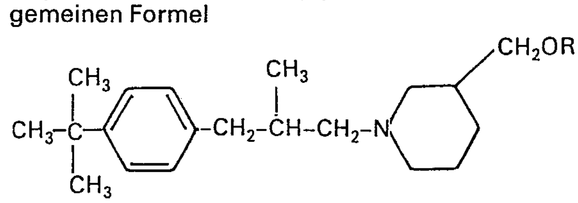

in der R einen Acetyl- oder Propionylrest bedeutet, und ihre Salze.

4. Verfahren zur Herstellung eines Fungizids, dadurch gekennzeichnet, dass man einen festen oder flüssigen Trägerstoff vermischt mit einem N-3′-(p-tertiär-Butylphenyl)-2′-methyl-propyl-1′-piperidinderivat der allgemeinen Formel

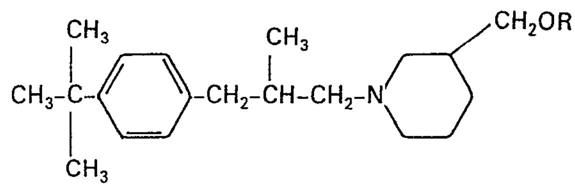

in der R einen Acetyl- oder Propionylrest bedeutet, und ihre Salze.

5. Verfahren zur Bekämpfung von Pilzen, dadurch gekennzeichnet, dass man die Pilze oder die vor Pilzbefall zu schützenden Gegenstände behandelt mit einem N-3′-(p-tertiär-Butylphenyl)-2′-methyl-propyl-1′-piperidinderivat der allgemeinen Formel

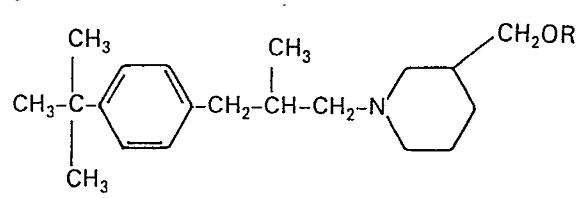

in der R einen Acetyl- oder Propionylrest bedeutet, und ihre Salze.

**Patentansprüche für den Vertragsstaat: AT**

1. Fungizid, enthaltend ein N-3′-(p-tertiär-Butylphenyl)-2′-methyl-propyl-1′-piperidinderivat der allgemeinen Formel

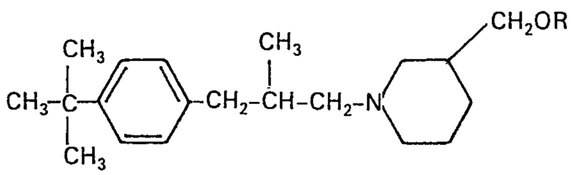

in der R einen Acetyl- oder Propionylrest bedeutet, oder seine Salze.

2. Fungizid, enthaltend einen festen oder flüssigen Trägerstoff und ein N-3′-(p-tertiär-Butylphenyl)-2′-methyl-propyl-1′-piperidinderivat gemäss Anspruch 1.

3. Verfahren zur Herstellung eines Fungizids, dadurch gekennzeichnet, dass man einen festen oder flüssigen Trägerstoff vermischt mit einem N-3′-(p-tertiär-Butylphenyl)-2′-methyl-propyl-1′-piperidinderivat gemäss Anspruch 1.

4. Verfahren zur Bekämpfung von Pilzen, dadurch gekennzeichnet, dass man die Pilze oder die vor Pilzbefall zu schützenden Gegenstände behandelt mit einem N-3′-(p-tertiär-Butylphenyl)-2′-methyl-propyl-1′-piperidinderivat gemäss Anspruch 1.

**Claims for the contracting states: BE, CH, LI, DE, FR, GB, IT, LU, NL, SE**

1. An N-(3′-(p-tert.-butylphenyl)-2′-methyl-prop-1′-yl)-piperidine derivative of the general formula

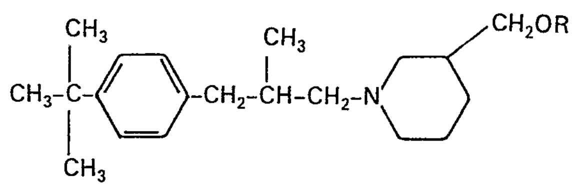

where R is acetyl or propionyl, or a salt thereof.

2. A fungicide containing an N-(3′-(p-tert.-butylphenyl)-2′-methyl-prop-1′-yl)-piperidine derivative of the general formula

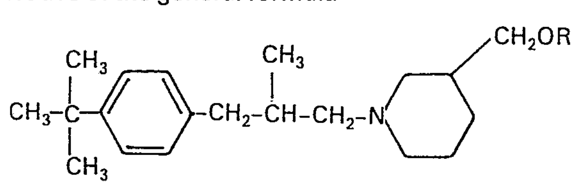

where R is acetyl or propionyl, or a salt thereof.

3. A fungicide containing a solid or liquid carrier and an N-(3′-(p-tert.-butylphenyl)-2′-methyl-prop-1′-yl)-piperidine derivative of the general formula

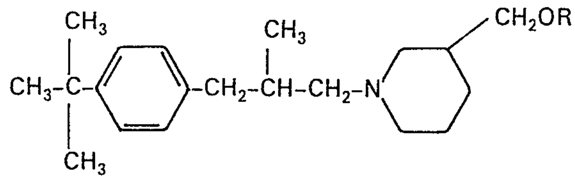

where R is acetyl or propionyl, or a salt thereof.

4. A process for producing a fungicide, wherein a solid or liquid carrier is mixed with an N-(3′-(p-tert.-butylphenyl)-2′-methyl-prop-1′-yl-)-piperidine derivative of the general formula

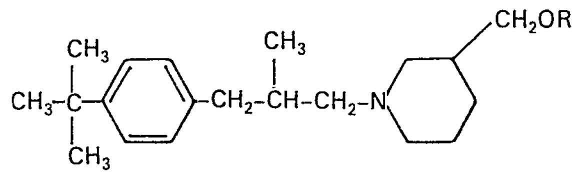

where R is acetyl or propionyl, or a salt thereof.

5. A process for combating fungi, wherein the fungi or the objects to be protected against fungus attack are treated with an N-(3′-(p-tert.-butylphenyl)-2′-methyl-prop-1′-yl)-piperidine derivative of the general formula

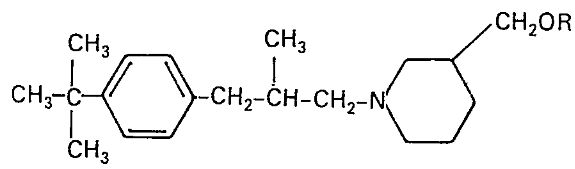

where R 1 is acetyl or propionyl, or a salt thereof.

**Claims for the contracting state: AT**

1. A fungicide containing an N-(3′-(p-tert.-butylphenyl)-2′-methyl-prop-1′-yl)-piperidine derivative of the general formula

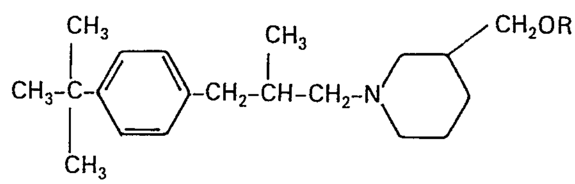

where R is acetyl or propionyl, or a salt thereof.

2. A fungicide containing a solid or liquid carrier and an N-(3′-(p-tert.-butylphenyl)-2′-methyl-prop-1′-yl)-piperidine derivative as defined in claim 1.

3. A process for producing a fungicide, wherein a solid or liquid carrier is mixed with an N-(3′-(p-tert.-butylphenyl)-2′-methyl-prop-1′-yl)-piperidine derivative as defined in claim 1.

4. A process for combating fungi, wherein the fungi or the objects to be protected against fungus attack are treated with an N-(3′-(p-tert.-butylphenyl)-2′-methyl-prop-1′-yl)-piperidine derivative as defined in claim 1.

**Revendications pour les états contractants: BE, CH, LI, DE, FR, GB, IT, LU, NL, SE**

1. Dérivé de N-3′-(p-tert-butylphényl)-2′-méthyl-propyl-1′-pipéridine de formule générale

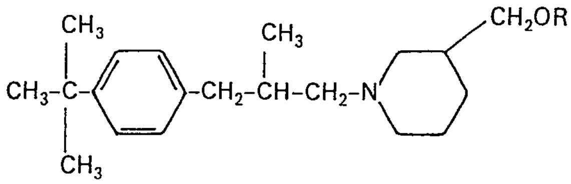

dans laquelle R représente un reste acétyle ou propionyle, et ses sels.

2. Fongicide contenant un dérivé de N-3′-(p-tert-butylphényl)-2′-méthyl-propyl-1′-pipéridine de formule générale

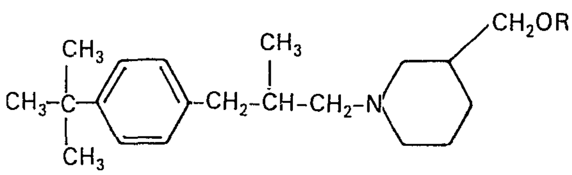

dans laquelle R représente un reste acétyle ou propionyle, et ses sels.

3. Fongicide contenant un véhicule solide ou liquide et un dérivé de N-3′-(p-tert-butylphényl)-2′-méthyl-propyl-1′-pipéridine de formule générale

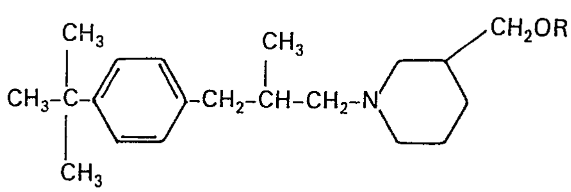

dans laquelle R représente un reste acétyle ou propionyle, et ses sels.

4. Procédé de préparation d'un fongicide, caractérisé par le fait que l'on mélange un véhicule solide ou liquide avec un dérivé de N-3′-(p-tert-butylphényl)-2′-méthyl-propyl-1′-pipéridine de formule générale

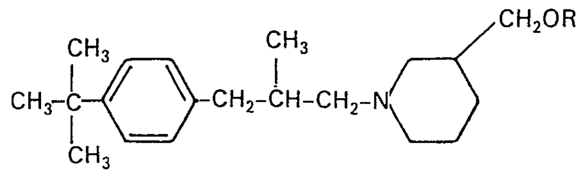

dans laquelle R représente un reste acétyle ou propionyle, et ses sels.

5. Procédé de lutte contre les champignons, caractérisé par le fait que l'on traite les champignons ou les objets à protéger contre l'attaque des champignons avec un dérivé de N-3′-(p-tert-butylphényl)-2′-méthyl-propyl-1′-pipéridine de formule générale

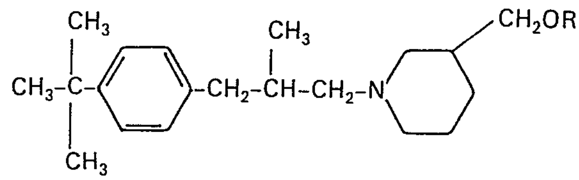

dans laquelle R représente un reste acétyle ou propionyle, et ses sels.

**Revendications pour l'état contractant: AT**

1. Fongicide contenant un dérivé de N-3′-(p-tert-butylphényl) 2′-méthyl-propyl-1′-pipéridine de formule générale

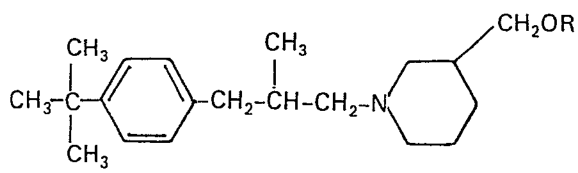

dans laquelle R représente un reste acétyle ou propionyle, et ses sels.

2. Fongicide contenant un véhicule solide ou liquide et un dérivé de N-3′-(p-tert-butylphényl)-2′-méthyl-propyl-1′-pipéridine selon la revendication 1.

3. Procédé de préparation d'un fongicide, caractérisé par le fait que l'on mélange un véhicule solide ou liquide avec un dérivé de N-3′-(p-tert-butylphényl)-2′-méthyl-propyl-1′-pipéridine selon la revendication 1.

4. Procédé de lutte contre les champignons, caractérisé par le fait que l'on traite les champignons ou les objets à protéger contre l'attaque des champignons avec un dérivé de N-3′-(p-tert-butylphényl)-2′-méthyl-propyl-1′-pipéridine selon la revendication 1.